# EUROPEAN PATENT SPECIFICATION

(11) **EP 2 574 357 B1**
(45) Date of publication and mention of the grant of the patent: **13.11.2013**
(21) Application number: 11183169.9
(22) Date of filing: 28.09.2011
(51) Int. Cl.: A61M 5/20, A61M 5/31, A61M 5/24

(54) **Electronic injector**
Elektronischer Injektor
Injecteur électronique

(43) Date of publication of application: 03.04.2013
(73) Proprietor: Q-Med AB, 752 28 Uppsala (SE)
(72) Inventor: Törnsten, Jonas, 752 28 Uppsala (SE); Blomqvist, Max, 745 40 Uppsala (SE); Dolk, Jonas, 167 14 Bromma (SE); Himbert, Hans, 167 14 Bromma (SE)
(74) Representative: Nilsson, Lars

(56) References cited:
- EP-A1- 2 361 647
- EP-A2- 2 351 590
- WO-A1-98/55168
- WO-A1-02/051471
- WO-A1-2010/145908
- US-A- 4 212 298
- US-A- 6 156 008
- US-A1- 2009 299 328

## Description

### FIELD OF THE INVENTION

The present invention relates to an injection device for delivering liquid compositions, such as viscous gels of e.g. hyaluronic acid.

### BACKGROUND OF THE INVENTION

WO2008/020023 discloses an injection device for injection of a predetermined quantity of a medicament. That injection device comprises a drive device for pushing a piston within a cartridge containing the medicament such that said quantity is expelled from the cartridge through an opening of a needle provided at one end of the cartridge. The drive device of that prior art document includes an energy accumulating member in the form of a spring which is wounded in advance by means of a tensioning knob. In order to expell the predetermined quantity, a user seizes the device similar to a pen and pushes a button provided near a front end of the device with his or her index finger. Even though that device has obvious advantages over normal normal handheld syringes, it has drawbacks concerning user ergonomics. For example, the construction of that device makes its rear end comparatively heavy which can be strenuous when the device is used for longer periods of time. Furthermore, there is always a risk that the accumulated energy will not suffice for the complete duration of the treatment which creates an unnecessary interruption of the treatment. EP-2351590 and US-2009/0299328 describe electronic injectors corresponding to the preamble of claim 1.

### SUMMARY OF THE INVENTION

It is an object of the present invention to provide an improved injection device which reduces or eliminates the above mentioned and other drawbacks. This object and other objects are achieved by an injection device according to the present invention as defined in claim 1 of the appended claims. This object and other objects are also achieved by an injection system as defined in claim 13 of the appended claims. Preferred embodiments of the present invention are defined in the dependent claims.

Thus, in accordance with an aspect of the present invention there is provided an injection device for delivering a liquid composition, comprising a generally elongated housing having first and second ends. The housing is adapted to hold an exchangeable cartridge comprising the liquid composition to be delivered proximal to said first end of said housing. A drive mechanism is arranged within the housing and comprises an electric motor and a power source. The motor is coupled to a plunger rod for acting on a cartridge received in said housing such that the liquid composition contained in the cartridge can be expelled. In order to activate the drive mechanism, a first actuation means is provided on an upper side of the housing, proximal to said first end of the housing and a second actuation means is provided at said second end of the housing, whereby the first and second actuating means are arranged to actuate the drive mechanism independent from each other. The electric motor and the power source can be provided within the housing such that the centre of gravity of the injection device is located at an appropriate position for obtaining good ergonomics. Due to the fact that actuation means are provided at two different positions of the injection device, a user can hold the device in a position similar to how a pen is held but also in a position similar to how a traditional hand-held syringe is held. Especially the latter position is preferred among many users since they are used to work with traditional hand-held syringes in this way. Furthermore, by using an electric motor, the working operation of winding the energy accumulating member in the form of a spring is avoided.

In accordance with an embodiment of the injection device of the invention, the power source comprises a rechargeable battery. A rechargeable battery require less space than the spring described in W02008/020023 and can be obtained in different sizes and different shapes. This results in a possibility of obtaining a favourable centre of gravity since a rechargeable battery can be positioned within the housing in many ways and that considerations of the space available within the housing can be attended to.

In accordance with an embodiment of the injection device of the invention, contact plates are provided on the housing through which the rechargeable battery can be connected to a charging station for recharging. This has the advantage that no male/female cable connections have to be provided on the injection device. Such connections are prone to cause loose contact hampering the charging of the battery. Further, such connections are often difficult to clean leading to residues and corrosion thereby impairing hygiene as well as reliable connection between the device and charger.

In accordance with an embodiment of the injection device of the invention, finger grips are provided on opposite first and second side walls of the elongated housing. The finger grips facilitate the operation of the injection device when it is held as a traditional hand-held syringe.

In accordance with an embodiment of the injection device of the invention, the drive means is capable of exerting a force of up to about 100 N on the liquid composition contained in a cartridge received in the housing. The required force depends on a wide range of parameters, such as the type of liquid composition to be delivered, the resistance provided by tissue at the location of delivery and the dimensions of the injection system (needle gauge, plunger diameter etc.). Therefore, it is necessary for the drive means to be able to exert a force on the liquid composition within a wide range. Typically viscous gels of e.g. hyaluronic acid requires a force of about 30-40 N.

In accordance with an embodiment of the injection device of the invention, the first and second actuation means comprise encapsulated switches. Such switches assure good hygiene since they are easy to keep clean.

In accordance with an embodiment of the injection device of the invention, at least one of the first and second actuation means comprises tactile feedback means for providing, for example, indications to a user about the status of an ongoing injection. A tactile feedback means can be very useful in practice since it allows for a user to continuously receive information from the injection device.

In accordance with an embodiment of the injection device of the invention, at least one of the first and second actuation means comprises a touch switch. A touch switch can be made to comply with even higher hygiene standards since it can be mounted within the housing of injection device and no moving parts or sealings are required.

In accordance with an embodiment of the injection device of the invention, means are provided for obtaining and presenting information to a user pertaining to an inserted cartridge. This reduces the risk that a cartridge comprising the wrong type of liquid composition is introduced into the device, thereby enhancing patient safety.

In accordance with an embodiment of the injection device of the invention, the housing is made from plastics. Plastics is convenient since it is easy and cost-effective to produce in any desired shape and it can be produced to comply with tough hygienic limits.

In accordance with an embodiment of the injection device of the invention, parts of the outer surface of the housing are provided with grip enhancing surface coating. This is convenient in order to avoid that a user slips when using the injection device, possibly causing pain and distress to a patient.

In accordance with another aspect of the invention, an injection system for delivering a liquid composition is provided, comprising an injection device according to any of the preceding claims, an exchangeable cartridge containing the liquid composition, and an injection needle attached to said cartridge, wherein said cartridge is held by said housing.

In accordance with an embodiment of the injection system of the invention, the exchangeable cartridge comprises finger grips. This facilitates for a user to hold the injection system in a syringe-like position.

In accordance with an embodiment of the injection system of the invention, the diameter of the injection needle ranges from 7 to 32 gauge.

Further objects and advantages of the present invention will be discussed below by means of exemplifying embodiments. These and other features, aspects and advantages of the invention will be more fully understood when considered with respect to the following detailed description, appended claims and accompanying drawings.

### BRIEF DESCRIPTION OF THE DRAWINGS

The invention will now be described in more detail and with reference to the appended drawings in which:
Figs. 1 and 2 are schematic perspective views of an embodiment of the injection system according to the invention.
Fig. 2a is a schematic perspective views of an embodiment of the injection system according to the invention when held by a user in a pen-like position.
Fig. 2b is a schematic perspective views of an embodiment of the injection system according to the invention when held by a user in a syringe-like position.
Fig. 2c is a schematic perspective view of an embodiment of the injection system according to the invention when held in an alternative position.
Fig. 3 is a schematic perspective view of a number of injection systems positioned in a rack.

### DESCRIPTION OF PREFERRED EMBODIMENTS

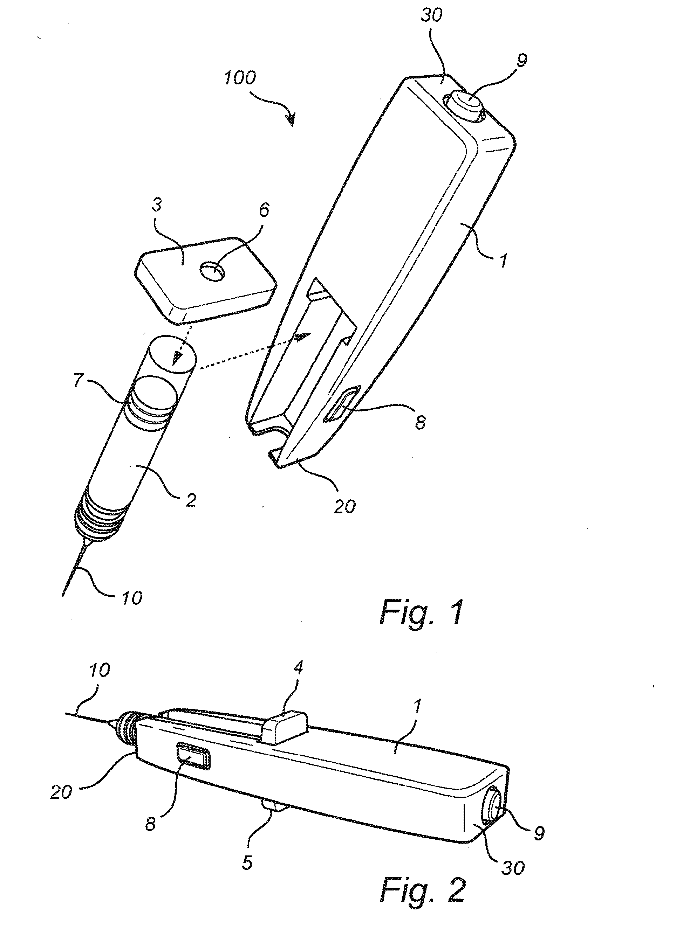

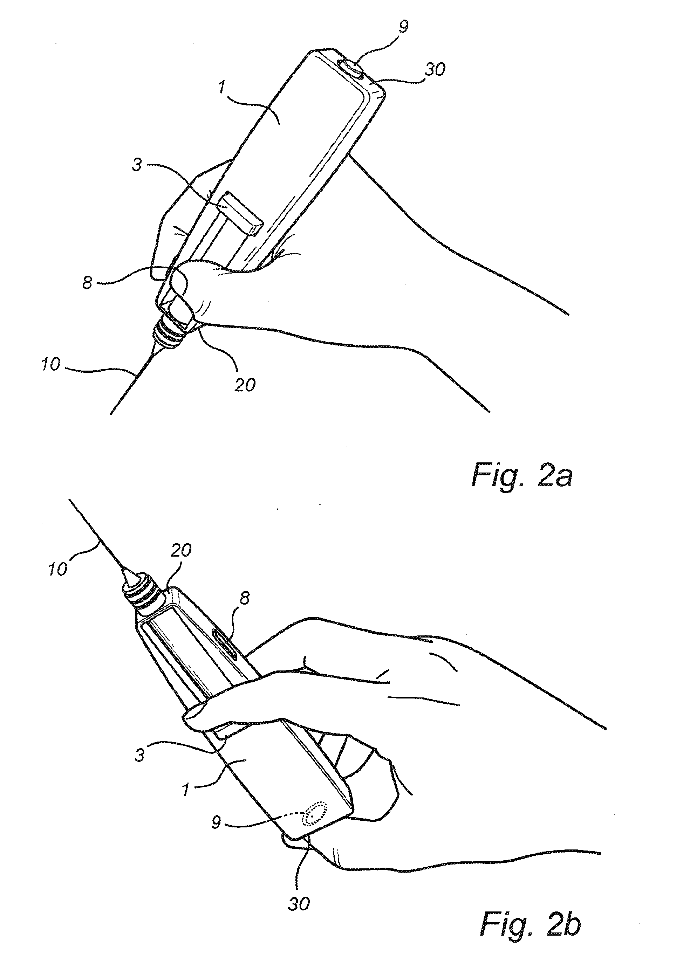

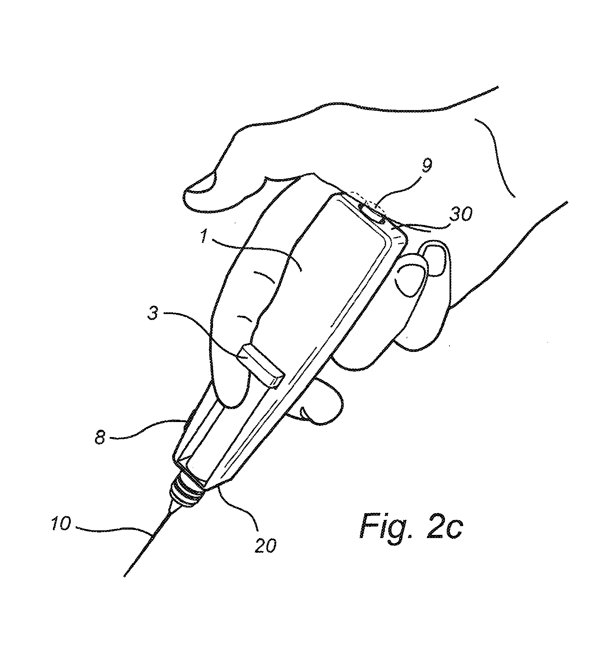

In a first embodiment of a injection system according to the invention, as shown in figures 1 and 2, an injection system 100 comprises a housing 1 made from thermoplastic and a cartridge 2. As used herein, the term "cartridge" encompasses all types of containers suitable for injectable liquid compositions, including gels. Further, the cartridge may comprise a rigidly mounted needle or a needle may be arranged, e.g. by threading, at one end of the cartridge. As shown in figure 1, the cartridge 2 can be inserted and firmly held within the housing 1. In this embodiment, the cartridge 2 is provided with a separate plate 3 providing finger grips 4 and 5. The plate 3 can be slipped onto the cartridge 2 and they may be attached to each other by means of a snap-connection in order to ensure a reliable coupling between the two parts. Reliable coupling between the cartridge 2 and the plate 3 can also be obtained by providing a press fit between the parts. The plate 3 and the cartridge 2 may of course also be integrally formed. It is also possible to include the finger grips in the housing 1. This has the advantage that finger grips are provided to a user whether finger grips are provided on the cartridge 2 or not since the provision of finger grips on one of the part does not exclude the provision of finger grips on the other. The cartridge 2 together with the plate 3 is inserted in the housing 1 as shown in figure 2 and is being firmly held in this position by means of a snap-connection or a press fit or even a separate locking member. When the cartridge 2 is correctly positioned in the housing, a plunger rod, not shown in the figures, driven by a motor of the injection device 100, also not shown in the figures, extends through the opening 6 in the plate 3 into the cartridge 2, thus engaging with the plunger 7 provided within the cartridge 2. In one embodiment, the plunger rod is driven by the motor through a threaded connection, see also further below, and consequently it performs its rectilinear motion under rotation. In this case, a spacer piece can be provided at the leading end of the plunger rod to reduce friction between plunger rod and plunger. The spacer piece is preferably rotatably journalled at the end of the plunger rod to avoid that the rotational motion acts upon the plunger. The spacer piece is preferably manufactured from a low friction material to reduce losses due to friction between itself and the plunger rod. The plunger rod can be driven forwards by a user by pushing one of the actuation means 8, 9, shown in the figures as encapsulated switches. It should be noted in this matter, that the actuation means are by no means restricted to encapsulated switches. The skilled person readily realizes that many other types of switches may be applied, such as touch switches and membrane switches. Tactile switches which respond differently depending on the force applied to it by a user are also conceivable. For example, a high depressing force on the switch trigger could cause the injection device 100 to expel the liquid composition at a higher rate compared to when a lower force is applied. The switches can also utilize haptic technology, i.e. taking advantage of a user's sense of touch by applying forces, vibrations, and/or motions to the user, such that feedback is provided to a user through the switch. It would thus be possible to have feedback through the switch regarding a number of parameters concerning the injection process, for example the expelling rate of the liquid composition and the resistance against which the liquid composition is expelled. It would also be possible to inform a user by haptics that the amount of liquid composition within the cartridge 2 is drawing towards an end or that there are compatibility problems between the cartridge 2 and the housing 1 due to faulty installation or due to installation of a cartridge 2 which is not intended for use with the injection device 100. Instead of haptics, or as a complement thereto, information may also be displayed on an LCD-screen, not shown in the figures, provided on the housing 1.

It would be possible to provide the injection device 100 with an RFID-reader capable of reading RFID-tags provided on the cartridges 2. These tags can provide information to the injection device 100 about the content, volume, date of manufacture etc of the cartridge 2 and its content. The information could be displayed on an LCD-screen provided on the housing 1 or presented to the user by haptics. It would also be possible to arrange a safety circuit within the device 100 to prevent the use of the device in cases were cartridges 2 with no or with defect RFID-tags or were the RFID-tags indicates that the content of the cartridge 2 is not intended for use with the injection device 100 in question. This greatly enhances safety of the patients.

The injection device 100 can be constructed to have the plunger rod travelling a predetermined distance when a switch 8, 9 is depressed. This means that a only predetermined amount of the liquid composition is expelled through the injection needle 10 each time a switch is actuated, independently from how long and how hard the switch 8, 9 is depressed. This facilitates for a user in situations where it is of a high importance that a maximum amount of the liquid composition is not exceeded. Another possibility is that the liquid composition is continuously expelled through the injection needle 10 as long as the switch 8, 9 is actuated.

The injection device according to the invention is useful for injecting liquid compositions, in particular viscous liquid compositions, such as gel compositions. Although gels display behaviour similar to solids, they contain mostly liquid, e.g. water. Injection of certain liquid compositions, such as hydrogels of e.g. hyaluronic acid or similar polymers, having high viscosities and high elasticity, requires rather high forces, especially in combination with small needles 10. This might have the effect that small amounts of the liquid composition may drip from the needle 10 even after motion of the plunger is stopped. Liquid composition dripping on the patient's skin may cause irritation and discomfort and should be avoided. Obviously, the dripping is also a waste of liquid composition and should as such be avoided. In order to prevent such drooling of the liquid composition, it is suggested that not only will the motor stop upon release of the actuation means, but it will even retract the plunger rod a certain distance. Due to its elastic properties, plunger 7 will be slightly compressed when travelling forward through cartridge 2. When the motion of the plunger 7 comes to a stop, a decompression will occur, i.e. the plunger will expand slightly to assume its normal shape. In other known solutions, the plunger could only expand in a forward direction causing the gel composition to drip out of the needle 10. Further, it is possible that air gets entrapped within the cartridge/needle system. During forward movement of the plunger 7, this air will be compressed within cartridge 2 and similar to the above-mentioned problem with the elasticity of the plunger this could cause gel composition to drip out of needle 10 even though the plunger 7 has come to a stop within cartridge 2. With the construction according to the present invention, however, plunger 7 may instead expand resp. move backwardly without substantial resistance towards the retracted plunger rod, thus eliminating said drooling.

Within the injection device 100, a drive mechanism comprising a motor and a power source is contained. The power source could comprise any type of rechargeable battery, such as, but not limited to, nickel cadmium (Ni-Cd), nickel metal hydride (NiMh), lithium ion (Li-ion) or lithium ion polymer (Li-ion polymer). Due to good its relatively high energy density and the fact that it can be provided in almost any desired shape, the Li-ion polymer is perhaps the best suited type for this application. The motor is preferably a DC motor such as a brushless or stepper dc-motor. Other types of motors are also conceivable. In order to be able to use the device for the injection of hydrogels of e.g. hyaluronic acid or similar polymers, or similar products, it is necessary that the motor is powerful enough that a force of up to about 100 N can be exerted on the plunger and thereby on the gel composition. The motor may be connected to the plunger rod by means of any of a number of different gear arrangements. For example the plunger rod may be provided with an external thread along at least a part of its length and the motor is connected to the plunger rod through this external thread. In this embodiment, the plunger rod will perform its rectilinear motion under rotation. Another possible solution involves a worm gear driven by the motor and a set of cogs provided on the plunger rod. Upon actuation of the motor, the worm gear will rotate and thereby cause a rectilinear motion by the plunger rod. It would also be possible to connect the motor with cogs provided on the plunger rod through one or more cog wheels, also known as rack and pinion gearing.

The cartridge used with the device will typically be provided with an injection needle 10 having a size between 21-32G when used for injection of hyaluronic acid gels. The device could, however, be used in other fields of applications as well were injection needles of other sizes, such as 7-32G, come into question.

Figures 2a, 2b and 2c show three possible positions in which the device 100 according to the invention may be held. In figure 2a, a user holds the device 100 similar to how a pen is held. The switch 8 is arranged at such a position that the user can actuate it with her/his index finger. Since the rechargeable battery and to some extent also the motor can be arranged with a high degree freedom within the housing 1 of the device 100, the centre of gravity of the device can be located at a position which is favourable for a user. If the centre of gravity is located towards the rear end, i.e. near the switch 9, the device can be perceived as uncomfortable and strenuous when used over longer periods of time. This is a major advantage of the device according to the present invention over devices according to the known prior art.

Figure 2b shows the device when held in a second position, similar to how a traditional syringe is held. Many medical practitioners, plastic surgeons and other users are used to operate a traditional syringe and actually prefer to hold the device 100 like this rather than in the pen-like position. The device according to the prior art does, however, not allow a user to do so. This is a further main advantage of the device 100 according to the present invention over the prior art. In this second position, the user can actuate the device 100 by means of switch 9 by her/his thumb. Protruding finger grips are provided to a user by plate 3 provided at the cartridge 2. Alternatively, finger grips can be arranged at the housing 1. This has the advantage that cartridges can be used which do not have plate 3 as well. However, it is preferred that if finger grips are provided at the housing 1 they should not prevent the use of cartridges 2 having a plate 3. The housing 1 should be designed such that the finger grips of the housing and the plate 3 lie adjacent to each other when a cartridge 2 is received in the housing 1.

Figure 2c shows the device when held in a position similar to that of figure 2b. In this case instead of using his or her thumb, the user actuates the device 100 by his or her palm. This position is preferred by some users and is made possible by the device 100 according to the present invention.

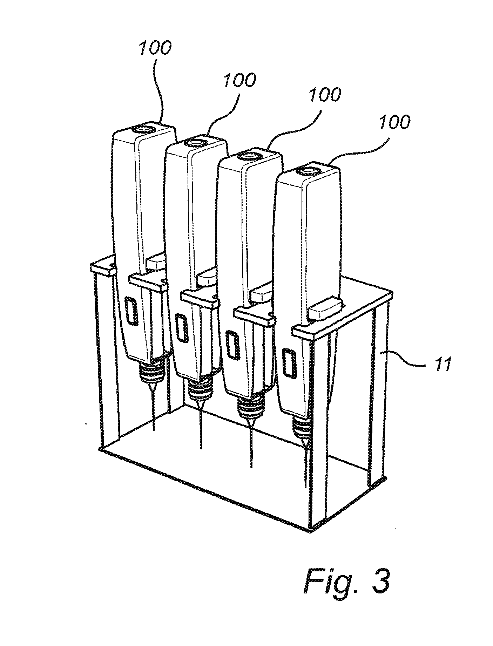

Figure 3 schematically shows a plurality of devices 100 suspended in a combined charging and storing rack 11. The rack 11 may comprise a charging station which can charge the rechargeable batteries of the devices 100 by means of conductive or inductive charging. Typically, contact plates are provided on the housing 1 of each device 100 for connecting the devices 100 to the charging station. This type of connection has a number of advantages in this case. For example, no male/female cable connections for charging have to be provided on the housing 1 and the charging station respectively. This, in turn, has a plurality of benefits to it such as simplified cleaning, avoidance of defective contacts, and improved water impermeability. Furthermore, in order to charge the device, a user simply puts the device in the rack 11. It is not necessary to connect a power cable, or similar, to the device.

Finally, it is realized, that an injection device according to the invention has a number of advantages over the known prior art devices. Examples are a more appropriate centre of gravity, no need for time consuming and tedious pre-tensioning of springs and the fact that the device can be held both similar to how a pen is held and similar to a traditional syringe, using either the thumb or the palm of the hand for actuation of the device. This freedom of choice of holding position is enabled by the provision of actuating means on an upper side of the elongated housing, proximal to the first end of the housing, as well as at the second end of the housing. This allows a user to actuate the device either with her/his index finger when used in a pen position or with her/his thumb or palm of the hand when used as a traditional syringe. The injection of crosslinked or non-crosslinked hyaluronic acid gels has been mentioned as a possible area of use for the device according to the invention. The hyaluronic acid gel is useful as a medical device, e.g. a dermal filler, for cosmetic use. It may also be useful in medical surgery, e.g. in eye surgery, joint surgery and medical cosmetic surgery or as a medicament, e.g. for treatment of joint disease. Naturally, it is possible to use the device according to the present invention with other liquid compositions, and preferably gel compositions, such as hydrogels. The device is also useful for injecting other types of dermal fillers than hyaluronic acid, e.g. collagen, calcium hydroxyl apatite, poly-L-lactic acid (PLLA), and polymethylmethacrylate (PMMA). Furthermore, the device is useful for injecting liquid compositions comprising active substances, e.g. bioactive agents, local anesthetics, cicatrizants, antioxidants or botulinum toxin. A preferred liquid composition of this type is a gel composition with a hyaluronic acid gel carrier and an active substance, e.g. a local anesthetic or a cictrizant, such as dextranomer beads.

## Claims

1. Injection device (100) for delivering a liquid composition, comprising
- a generally elongated housing (1) having first and second ends (20, 30);
- said housing (1) being adapted to receive an exchangeable cartridge (2), comprising said liquid composition, proximal to said first end (20) of said housing (1); and
- a drive mechanism arranged within said housing, said drive mechanism comprising an electric motor and a power source, wherein the motor is coupled to a plunger rod for acting on a cartridge (2) received in said housing (1) such that the liquid composition contained in the cartridge (2) can be expelled; and
- a first actuation means (8) for actuating the drive mechanism is provided on an upper side of the elongated housing (1), proximal to said first end (20) of the housing (1),
**characterized in that** a second actuation means (9) for actuating the drive mechanism is provided at said second end (30) of the housing (1), wherein said first and second actuating means are arranged to actuate the drive mechanism independent from each other.

2. Injection device (100) according to any of the preceding claims, wherein the power source comprises a rechargeable battery.

3. Injection device (100) according to claim 2, wherein contact plates are provided on the housing through which the rechargeable battery can be connected to a charging station for recharging.

4. Injection device (100) according to any of the preceding claims, wherein finger grips are provided on opposite first and second side walls of the elongated housing (1).

5. Injection device (100) according to any of the preceding claims, wherein the drive means is capable of exerting a force of up to about 100 N on the liquid composition contained in a cartridge (2) received in the housing (1).

6. Injection device (100) according to any of the preceding claims, wherein said first and second actuation means (8, 9) comprise encapsulated switches.

7. Injection device (100) according to any of the preceding claims, wherein at least one of the first and second actuation means (8, 9) comprises tactile feedback means for providing, for example, indications to a user about the status of an ongoing injection.

8. Injection device (100) according to any of the preceding claims, wherein at least one of the first and second actuation means (8, 9) comprises a touch switch.

9. Injection device (100) according to any of the preceding claims, wherein means are provided for obtaining and presenting information to a user pertaining to an inserted cartridge (2).

10. Injection device (100) according to any of the preceding claims, wherein the housing (1) is made from plastics.

11. Injection device (100) according to any of the preceding claims, wherein parts of the outer surface of the housing (1) is provided with grip enhancing surface coating.

12. Injection system for delivering a liquid composition, preferably a gel composition, comprising an injection device (100) according to any of claims 1 to 11, an exchangeable cartridge (2) containing said liquid composition, and an injection needle (10) attached to said cartridge (2), wherein said cartridge (2) can be held by said housing (1).

13. Injection system for delivering a liquid composition according to claim 12, wherein the exchangeable cartridge (2) comprises finger grips (4, 5).

14. Injection system for delivering a liquid composition according to claim 12 or 13, wherein the diameter of the injection needle (10) ranges from 7 to 32 gauge.

## Patentansprüche

1. Injektionsvorrichtung (100) für das Verabreichen einer flüssigen Zusammensetzung, die Folgendes aufweist:
- ein im Allgemeinen langgestrecktes Gehäuse (1) mit ersten und zweiten Enden (20, 30);
- wobei das Gehäuse (1) ausgebildet ist, eine auswechselbare Kartusche (2), welche die flüssige Zusammensetzung enthält, proximal zu dem ersten Ende (20) des Gehäuses (1) aufzunehmen; und
- einen Antriebsmechanismus, welcher innerhalb des Gehäuses angeordnet ist, der Antriebsmechanismus einen elektrischen Motor und eine Energiequelle umfasst, wobei der Motor zur Beaufschlagung einer Kartusche (2), welche in dem Gehäuse (1) aufgenommen ist, derart an eine Kolbenstange gekoppelt ist, dass die flüssige Zusammensetzung, die in der Kartusche (2) enthalten ist, ausgetrieben werden kann; und
- eine erste Betätigungseinrichtung (8), die zum Betätigen des Antriebsmechanismus auf einer Oberseite des langgestreckten Gehäuses (1) proximal zu dem ersten Ende (20) des Gehäuses (1) bereitgestellt ist,
**dadurch gekennzeichnet, dass** eine zweite Betätigungseinrichtung (9) zum Betätigen des Antriebsmechanismus an dem zweiten Ende (30) des Gehäuses (1) bereitgestellt ist, wobei die ersten und zweiten Betätigungseinrichtungen eingerichtet sind, den Antriebsmechanismus unabhängig voneinander zu betätigen.

2. Injektionsvorrichtung (100) gemäß einem der vorhergehenden Ansprüche, wobei die Energiequelle eine wiederaufladbare Batterie umfasst.

3. Injektionsvorrichtung (100) gemäß Anspruch 2, wobei Kontaktplättchen auf dem Gehäuse bereitgestellt sind, durch welche die wiederaufladbare Batterie mit einer Ladestation zum Aufladen verbunden werden kann.

4. Injektionsvorrichtung (100) gemäß einem der vorhergehenden Ansprüche, wobei Fingermulden auf gegenüberliegenden ersten und zweiten Seitenwänden des langgestreckten Gehäuses (1) bereitgestellt werden.

5. Injektionsvorrichtung (100) gemäß einem der vorhergehenden Ansprüche, wobei die Antriebseinrichtung in der Lage ist, eine Kraft von bis zu ungefähr 100 N auf die flüssige Zusammensetzung auszuüben, die in einer Kartusche (2) enthalten ist, welche in das Gehäuse (1) aufgenommen ist.

6. Injektionsvorrichtung (100) gemäß einem der vorhergehenden Ansprüche, wobei die ersten und zweiten Betätigungseinrichtungen (8, 9) gekapselte Schalter aufweisen.

7. Injektionsvorrichtung (100) gemäß einem der vorhergehenden Ansprüche, wobei mindestens eine der ersten und zweiten Betätigungseinrichtungen (8, 9) taktile Rückmeldungsmittel aufweist, um, zum Beispiel, Anzeigen über den Stand einer laufenden Injektion für einen Benutzer bereitzustellen.

8. Injektionsvorrichtung (100) gemäß einem der vorhergehenden Ansprüche, wobei mindestens eine der ersten und zweiten Betätigungseinrichtungen (8, 9) einen Berührungsschalter aufweist.

9. Injektionsvorrichtung (100) gemäß einem der vorhergehenden Ansprüche, wobei Mittel zum Erhalten und Darstellen von Informationen, die sich auf eine eingesetzte Kartusche (2) beziehen, für einen Benutzer bereitgestellt werden.

10. Injektionsvorrichtung (100) gemäß einem der vorhergehenden Ansprüche, wobei das Gehäuse (1) aus Kunststoff hergestellt ist.

11. Injektionsvorrichtung (100) gemäß einem der vorhergehenden Ansprüche, wobei Teilbereiche der Außenfläche des Gehäuses (1) mit einer die Griffigkeit verbessernden Oberflächenbeschichtung versehen sind.

12. Injektionssystem für die Verabreichung einer flüssigen Zusammensetzung, vorzugsweise einer Gelzusammensetzung, die eine Injektionsvorrichtung (100) gemäß einem der Ansprüche 1 bis 11, eine austauschbare Kartusche (2), welche die flüssige Zusammensetzung enthält, und eine Injektionsnadel (10) umfasst, die an der Kartusche (2) befestigt ist, wobei die Kartusche (2) durch das Gehäuse (1) gehalten werden kann.

13. Injektionssystem für die Verabreichung einer flüssigen Zusammensetzung gemäß Anspruch 12, wobei die austauschbare Kartusche (2) Fingermulden (4, 5) aufweist.

14. Injektionssystem für die Verabreichung einer flüssigen Zusammensetzung gemäß Anspruch 12 oder 13, wobei der Durchmesser der Injektionsnadel (10) in einem Bereich von 7 bis 32 Gauge liegt.

## Revendications

1. Dispositif d'injection (100) pour distribuer une composition liquide, comprenant
- un boîtier généralement allongé (1) ayant des première et seconde extrémités (20, 30) ;
- ledit boîtier (1) étant adapté pour recevoir une cartouche échangeable (2), comprenant ladite composition liquide, à proximité de ladite première extrémité (20) dudit boîtier (1) ;
- un mécanisme d'entraînement disposé à l'intérieur dudit boîtier, ledit mécanisme d'entraînement comprenant un moteur électrique et une source d'énergie, dans lequel le moteur est couplé à une tige de piston pour agir sur une cartouche (2) reçue dans ledit boîtier (1), de telle sorte que la composition liquide contenue dans la cartouche (2) puisse être expulsée, et
- un premier moyen d'actionnement (8) pour actionner le mécanisme d'entraînement est prévu sur un côté supérieur du boîtier allongé (1), à proximité de ladite première extrémité (20) du boîtier (1) ;
**caractérisé en ce qu'**un second moyen d'actionnement (9) pour actionner le mécanisme d'entraînement est prévu au niveau de ladite deuxième extrémité (30) du boîtier (1), dans lequel lesdits premier et deuxième moyens d'actionnement sont agencés pour actionner le mécanisme d'entraînement de manière indépendante.

2. Dispositif d'injection (100) selon l'une quelconque des revendications précédentes, dans lequel la source d'énergie comprend une batterie rechargeable.

3. Dispositif d'injection (100) selon la revendication 2, dans lequel des plaques de contact sont prévus sur le boîtier à travers lequel la batterie rechargeable peut être connectée à une station de recharge pour la recharge.

4. Dispositif d'injection (100) selon l'une quelconque des revendications précédentes, dans lequel les prises pour les doigts sont disposées sur les première et deuxième parois latérales opposées du boîtier allongé (1).

5. Dispositif d'injection (100) selon l'une quelconque des revendications précédentes, dans lequel le moyen d'entraînement est capable d'exercer une force d'environ maximum 100 N sur la composition liquide contenue dans une cartouche (2) reçue dans le boîtier (1).

6. Dispositif d'injection (100) selon l'une quelconque des revendications précédentes, dans lequel lesdits premier et deuxième moyens d'actionnement (8, 9) comprennent des commutateurs encapsulés.

7. Dispositif d'injection (100) selon l'une quelconque des revendications précédentes, dans lequel au moins l'un des premier et second moyens d'actionnement (8, 9) comprend des moyens pour fournir un retour tactile, par exemple, des indications à l'utilisateur sur l'état d'une injection continue.

8. Dispositif d'injection (100) selon l'une quelconque des revendications précédentes, dans lequel au moins l'un des premier et second moyens d'actionnement (8, 9) comprend un commutateur de contact.

9. Dispositif d'injection (100) selon l'une quelconque des revendications précédentes, dans lequel des moyens sont prévus pour l'obtention et la présentation d'informations se rapportant à une cartouche insérée à un utilisateur (2).

10. Dispositif d'injection (100) selon l'une quelconque des revendications précédentes, dans lequel le boîtier (1) est réalisé en matière plastique.

11. Dispositif d'injection (100) selon l'une quelconque des revendications précédentes, dans lequel des parties de la surface extérieure du boîtier (1) sont munies de revêtement de surface pour accroître l'adhérence.

12. Système d'injection pour distribuer une composition liquide, de préférence une composition de gel comprenant un dispositif d'injection (100) selon l'une quelconque des revendications 1 à 11, dans une cassette interchangeable (2) contenant ladite composition liquide, et une aiguille d'injection (10) fixée à ladite cartouche (2), dans lequel ladite cartouche (2) peut être maintenue par ledit logement (1).

13. Système d'injection pour distribuer une composition liquide selon la revendication 12,dans lequel la cassette interchangeable (2) comprend des prises pour les doigts (4, 5).

14. Système d'injection pour distribuer une composition liquide selon la revendication 12 ou 13, dans lequel le diamètre de l'aiguille d'injection (10) varie de 7 à 32.
